# EUROPEAN PATENT APPLICATION

(11) **EP 4 595 906 A1**
(43) Date of publication of application: **06.08.2025**
(21) Application number: 23871341.6
(22) Date of filing: 28.06.2023
(51) Int. Cl.: A61B 17/32

(54) **SURGICAL TREATMENT DEVICE**

(30) Priority: 30.09.2022 JP 2022158389
(71) Applicant: FUJIFILM Corporation, Tokyo 106-8620 (JP)
(72) Inventor: HIGASHI, Kohei, Ashigarakami-gun, Kanagawa 258-8577 (JP); AOSHIMA, Keisuke, Ashigarakami-gun, Kanagawa 258-8577 (JP); NAKAMURA, Koichiro, Ashigarakami-gun, Kanagawa 258-8577 (JP)
(74) Representative: HGF
(86) International application number: PCT/JP2023/023982
(87) International publication number: WO 2024/070096

(57) **Abstract**

Provided is a surgical treatment device that can seal a necessary part by self-heating in a biological tissue, such as a blood vessel, and accurately incise the sealed part via ultrasound oscillation. A surgical treatment device (20) includes a treatment tool including a transducer (25) including a piezoelectric element (39), an impedance matching circuit (44), a power supply wiring line, and a control mechanism (41) that controls an amount of power supplied, in which the transducer (25) is located at a distal end of the treatment tool and includes an opening/closing mechanism (32) that opens and closes grip pieces (30a) and (30b) that grip a biological tissue, the piezoelectric element (39) is installed on at least one of the grip piece (30a) or the grip piece (30b), a stress concentration structure (31) is provided on at least one of the grip piece (30a) or the grip piece (30b), and the piezoelectric element (39) is driven by using a first driving frequency in a case in which the biological tissue is sealed, and is driven by using a second driving frequency different from the first driving frequency in a case in which the biological tissue is incised.

## Description

### BACKGROUND OF THE INVENTION

### 1. Field of the Invention

The present invention relates to a surgical treatment device.

### 2. Description of the Related Art

In the medical field, it is known that various treatments are performed on a subject using a surgical treatment tool that generates ultrasound oscillation. JP2012-050845A describes that a composite ultrasound treatment system is used to control an ultrasound transducer at a first frequency for a low frequency treatment and a second frequency for a high frequency treatment, and biological reactions are started at each frequency. JP2017-127639A discloses an ultrasound surgical instrument that simultaneously generates a low frequency waveform and a high frequency waveform by an ultrasound mechanical motion generated in a probe, and seals and cuts a tissue.

### SUMMARY OF THE INVENTION

In the ultrasound treatment described in JP2012-050845A, both the first frequency and the second frequency are non-invasive treatments used for hemostasis or cauterization on the tissue of a target region, and there is no description of performing a treatment on a blood vessel or the like in the body. In addition, the first frequency is about 1 MHz or more and about 3 MHz or less, and the second frequency is about 3 MHz or more and about 100 MHz or less, but there is no specific description regarding a transducer that provides the first and second frequencies, and a dimension such as a thickness is unknown.

In an electrosurgical system described in JP2017-127639A, a generator for double output is used to simultaneously output a first frequency and a second frequency, and excision or coagulation of the tissue is performed. On the other hand, there is no description of regulations or the like regarding a member configuration, and a type, a shape, and the like of the member used are unknown. Therefore, there is no specific description regarding the excision of the tissue, and there is no specification regarding how to perform the excision. Further, since the two frequencies are output simultaneously, it is not possible to perform the two treatments at different timings, and thus there is a concern that hemostasis is insufficient and the blood vessel or the like is incised, or there is a concern that only hemostasis is performed and the incision of the excised part is not accurately performed.

There is a method of performing, on a tissue such as the blood vessel in which contents leak or overflow in a case in which the tissue is incised as it is, a sealing treatment such as hemostasis or coagulation by converting a voltage of an ultrasound frequency into heat by applying the voltage to a device, and incising a soft tissue such as a blood vessel by converting the voltage of the ultrasound frequency into oscillation by applying the voltage to the device to a part subjected to the sealing treatment. In this case, it is required to accurately incise the part in which the sealing treatment is performed while minimizing damage to the biological tissue in a vicinity of a treatment portion due to the spread of heat without entraining an unsealed portion.

An object of the present invention is to provide a surgical treatment device that can seal a necessary part by the self-heating in a biological tissue, such as a blood vessel, and accurately incise the sealed part via ultrasound oscillation.

An aspect of the present invention relates to a surgical treatment device comprising: a treatment tool including a transducer including a piezoelectric element; an impedance matching circuit for driving the piezoelectric element; a power supply wiring line that supplies power to the piezoelectric element; and a control mechanism that controls an amount of the power supplied to the piezoelectric element, in which the transducer is located at a distal end of the treatment tool and includes an opening/closing mechanism that opens and closes grip pieces that grip a biological tissue, the piezoelectric element is installed on at least one of the grip pieces, a stress concentration structure is provided on at least one of the grip pieces, the impedance matching circuit drives the piezoelectric element by using a first driving frequency and a second driving frequency that are frequencies different from each other, and the piezoelectric element is driven by using the first driving frequency in a case in which the biological tissue is sealed, and is driven by using the second driving frequency in a case in which the biological tissue is incised.

It is preferable that the first driving frequency is a resonance frequency derived from a thickness dimension of the piezoelectric element, and the second driving frequency is a resonance frequency derived from a length dimension of the piezoelectric element.

It is preferable that a frequency of the first driving frequency is in a range of 1 MHz or more and 10 MHz or less, and a frequency of the second driving frequency is in a range of 1 kHz or more and 1 MHz or less.

It is preferable that power supply at the first driving frequency is executed in a first set period in response to detection of the grip of the transducer.

It is preferable that power supply at the second driving frequency is executed in a second set period, and the grip is released in response to an end of the second set period.

It is preferable that switching from the first driving frequency to the second driving frequency is executed in a state in which the grip is maintained.

It is preferable that the switching from the first driving frequency to the second driving frequency is automatically executed in response to an end of power supply at the first driving frequency.

It is preferable that pressurization is performed during power supply at the first driving frequency, and a grip strength of the transducer is stronger than a grip strength at a start of the grip.

It is preferable that a grip strength of the transducer is controlled in a stepwise manner by a manual operation of a user.

It is preferable that the stress concentration structure is linearly disposed from a vicinity of a distal end of the transducer toward the opening/closing mechanism, and is parallel to a longitudinal direction of the transducer.

According to the aspect of the present invention, it is possible to seal the necessary part by the self-heating in the biological tissue, such as the blood vessel, and accurately incise the sealed part via the ultrasound oscillation.

### BRIEF DESCRIPTION OF THE DRAWINGS

Fig. 1A is an explanatory diagram of a configuration of an endoscope system, and Fig. 1B is an explanatory diagram of a configuration of a surgical treatment device.
Fig. 2 is an explanatory diagram of a state in which a surgical treatment tool is inserted into an endoscope.
Fig. 3 is a perspective view of a state (A) in which grip pieces of the surgical treatment tool are closed and a state (B) in which the grip pieces are opened.
Fig. 4 is a cross-sectional view of a state (A) in which the grip pieces of the surgical treatment tool are closed and a state (B) in which the grip pieces are opened.
Fig. 5 is a cross-sectional view taken along a line IV-IV of (A) of Fig. 4.
Fig. 6 is a schematic diagram of a piezoelectric element constituting an ultrasound oscillator.
Fig. 7 is an explanatory diagram of a stress concentration structure.
Fig. 8 is a block diagram illustrating an outline of the surgical treatment device.
Fig. 9 is a flowchart illustrating the series of flows of blood vessel sealing and incision.

### DESCRIPTION OF THE PREFERRED EMBODIMENTS

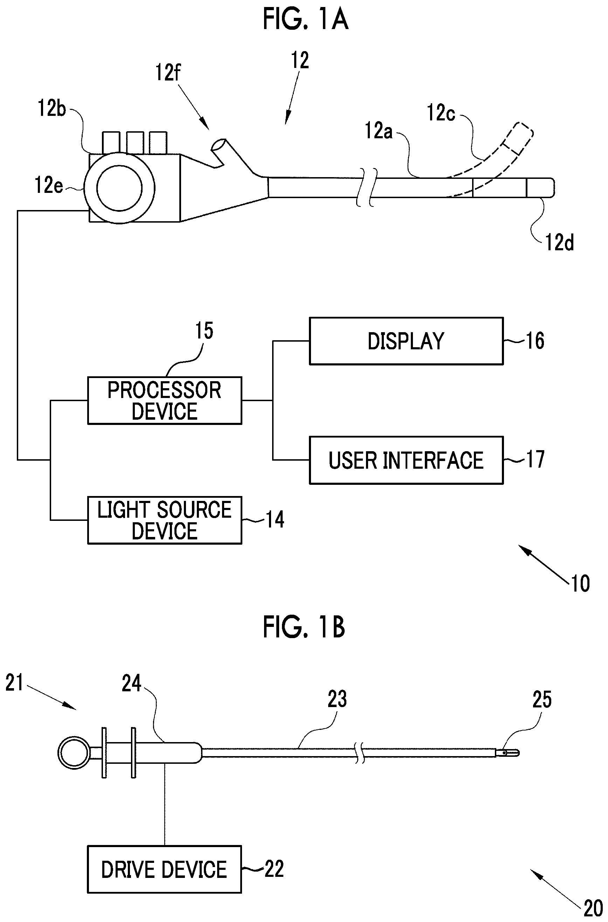

Figs. 1A and 1B include Fig. 1A illustrating an endoscope system 10 used in combination with a surgical treatment device 20 and Fig. 1B illustrating the surgical treatment device 20 including a surgical treatment tool 21. As illustrated in Fig. 1A, the endoscope system 10 comprises an endoscope 12, a light source device 14, a processor device 15, a display 16, and a user interface (UI) 17. The endoscope 12 captures an image of an observation target. The light source device 14 emits illumination light for irradiating the observation target. The processor device 15 performs system control of the endoscope system 10. The display 16 is a display unit that displays an observation image and the like based on an endoscope image. The user interface (UI) 17 is an input device that performs setting input and the like to the processor device 15 and the like, such as a mouse and a keyboard.

The endoscope 12 is optically connected to the light source device 14 and electrically connected to the processor device 15. The endoscope 12 has an insertion part 12a to be inserted into a subject, an operating part 12b provided at a base end part of the insertion part 12a, a bendable part 12c provided on a distal end side of the insertion part 12a, and a distal end part 12d. The bendable part 12c is bent by operating an angle knob 12e of the operating part 12b. As a result, the distal end part 12d is directed in a desired direction. Further, a forceps port 12f is provided in the operating part 12b in addition to the angle knob 12e. The forceps port 12f is an inlet for inserting an endoscopic treatment tool such as the surgical treatment tool 21. The endoscope treatment tool is used in a state of being inserted into the forceps port 12f. The endoscope 12 may be either a flexible endoscope or a rigid endoscope.

A distal end surface of the distal end part 12d is provided with an observation window or an illumination window. An image sensor (not illustrated) or the like is disposed behind the observation window, and an optical fiber cable (not illustrated) is disposed behind the illumination window. The signal line of the image sensor and the optical fiber cable is connected to each of the processor device 15 and the light source device 14.

The processor device 15 is electrically connected to the display 16 and the user interface 17. The processor device 15 performs image processing and the like on the endoscope image captured by the image sensor, and displays the processed endoscope image on the display 16.

As illustrated in Fig. 1B, the surgical treatment device 20 comprises the surgical treatment tool 21, which is one of endoscopic treatment tools inserted into the subject through the endoscope 12, and a drive device 22 that supplies power. The surgical treatment tool 21 includes a flexible tubular sheath 23, an operating part 24, and a transducer 25, and is electrically connected to the drive device 22. The flexible tubular sheath 23 is a tubular sheath made of a flexible material, for example, a soft resin, and is inserted into a forceps channel 12g of the endoscope 12. The surgical treatment device 20 including the surgical treatment tool 21 is used, for example, for sealing and incision in a case of laparoscopic surgery. The transducer 25 is located at a distal end of the surgical treatment tool 21 and grips a biological tissue. Specifically, the biological tissue to be gripped is a soft tissue such as a blood vessel. The operating part 24 receives a user operation of opening and closing the transducer 25 or adjusting a grip strength in a state in which the transducer 25 is closed, that is, in a gripping state.

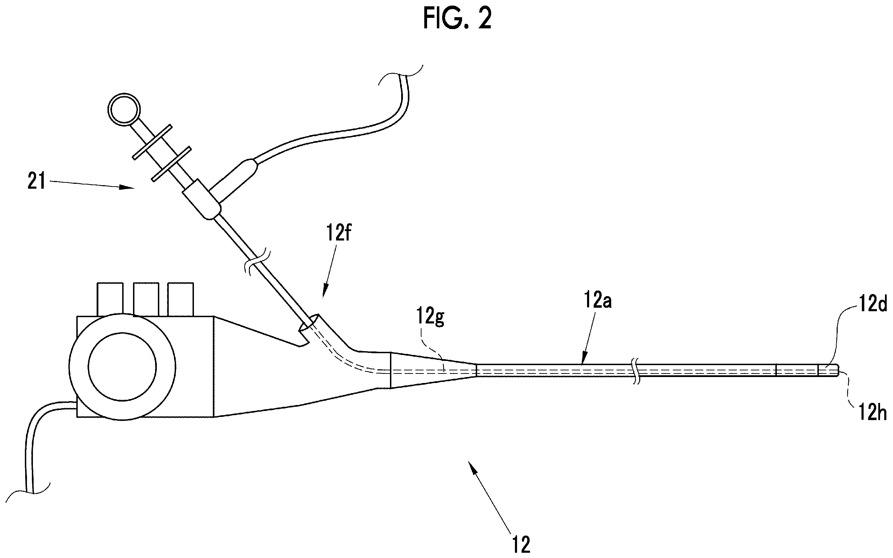

As illustrated in Fig. 2, the forceps channel 12g for inserting the surgical treatment tool 21 is disposed in the insertion part 12a having the distal end part 12d. The transducer 25 protrudes from a forceps outlet 12h of the distal end part 12d in the surgical treatment tool 21 inserted into the forceps port 12f. One end of the forceps channel 12g is connected to the forceps outlet 12h, and the other end thereof is connected to the forceps port 12f provided in the operating part 12b. In addition, the forceps channel 12g is also used as a path for feeding a washing solution such as water from the forceps outlet 12h and a path for suctioning a body fluid such as blood and contents such as body waste materials. The drive device 22 supplies the power to the surgical treatment tool 21.

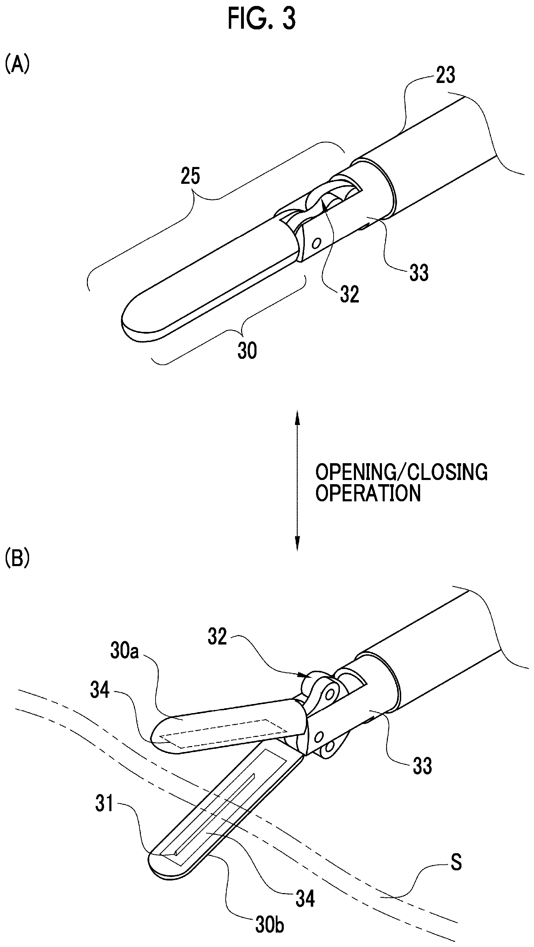

As illustrated in Fig. 3, the transducer 25 includes a grip part 30 having a pair of grip pieces 30a and 30b that can be opened and closed in an up-down direction, an opening/closing mechanism 32 that opens and closes the grip part 30, and a support member 33 that supports the grip part 30 and the opening/closing mechanism 32. Further, the flexible tubular sheath 23 partially includes the support member 33. (A) of Fig. 3 illustrates a state in which the grip part 30 is closed, and (B) of Fig. 3 illustrates a state in which the grip part 30 is opened. The grip pieces 30a and 30b are each formed in a semi-cylindrical shape, and the transducer 25 including the grip part 30 in a closed state has a cylindrical shape and at least a shape equal to or smaller than the circumference of the flexible tubular sheath 23. As illustrated in (B) of Fig. 3, acoustic matching layers 34 are provided on inner surfaces of the pair of grip pieces 30a and 30b and surfaces facing each other. The opening and closing of the grip part 30 is the opening and closing of the transducer 25. In addition, in a state in which the transducer 25 is closed to interpose a structure S, such as the blood vessel, a stress concentration structure 31 in which stress is concentrated is provided on an inner surface of at least one of the grip piece 30a and the grip piece 30b. Specifically, the stress concentration structure 31 is disposed on a surface of the acoustic matching layer 34.

In a case in which the transducer 25 is in an opened state, the structure S, which is the biological tissue such as the blood vessel, is passed between the grip piece 30a and the grip piece 30b, and the transducer 25 is closed, so that the inner surfaces of the pair of grip pieces 30a and 30b are brought close to each other, and the structure S can be gripped by being interposed between the pair of grip pieces 30a and 30b. In this way, the structure S, such as the blood vessel, which is a treatment target, such as sealing, is interposed by the grip part 30 consisting of the grip pieces 30a and 30b. A large amount of water is contained in the soft tissue such as the blood vessel, which is a factor that delays an increase in temperature. Therefore, the appropriate grip (pressurization) of the structure S pushes out the moisture contained in the biological tissue and promotes the denaturation or adhesion of the remaining biological tissue, thereby enhancing the sealing effect. It should be noted that, in the present treatment tool, an appropriate grip pressure at which the sealing effect can be obtained is 10 to 50 N.

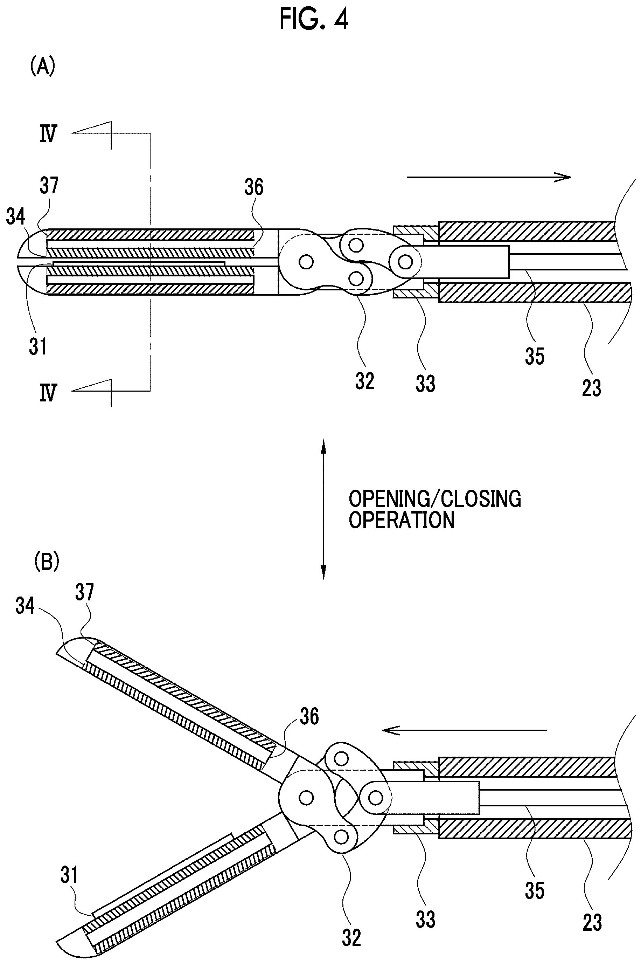

Fig. 4 is a cross-sectional view in the vicinity of the transducer 25, and (A) of Fig. 4 illustrates a cross-sectional view in a state in which the transducer 25 is closed, and (B) of Fig. 4 illustrates a cross-sectional view in a state in which the transducer 25 is opened. An operation wire 35 is inserted into the flexible tubular sheath 23. The operation wire 35 transmits an opening/closing operation performed the user using the operating part 24 of the surgical treatment tool 21 to the opening/closing mechanism 32. In the opening/closing operation, the operation wire 35 is pulled by the flexible tubular sheath 23 to obtain the closed state of the opening/closing mechanism 32, and is pushed to the distal end part to obtain the opened state of the opening/closing mechanism 32. It should be noted that, in the closed state, since at least one of the grip pieces 30a or 30b is provided with the stress concentration structure 31, a gap corresponding to a height of the stress concentration structure 31 is generated without being in close contact.

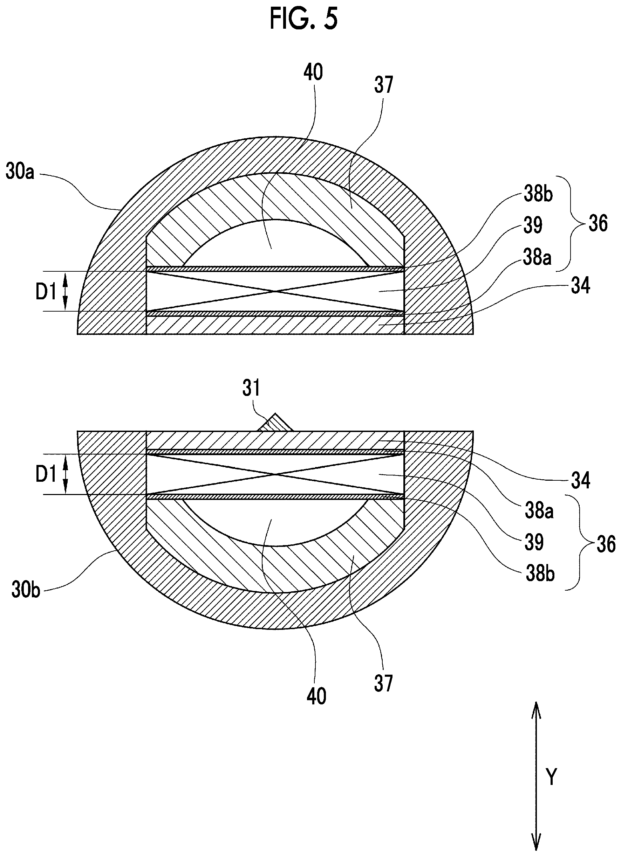

In the grip piece 30a and the grip piece 30b, the acoustic matching layer 34, the ultrasound oscillator 36, and the backing member 37 are embedded in the inner surfaces that interpose the structure S. By using ultrasound oscillation caused by the ultrasound oscillator 36, the temperature of the structure S such as the blood vessel is raised, and the structure S is sealed. One surface of the ultrasound oscillator 36 is fixed to a transducer housing with a backing member 37 interposed therebetween, and the other surface is covered with an insulating layer and/or the acoustic matching layer 34. The cross-sectional view taken along a line IV-IV of (A) of Fig. 4 will be described with reference to Fig. 5.

As illustrated in Fig. 5, the acoustic matching layer 34 and the ultrasound oscillator 36 are respectively laminated in parallel on the grip pieces 30a and 30b of the transducer 25. The backing member 37 is made of a material having rigidity, such as hard rubber, and supports the ultrasound oscillator 36 from the grip piece side that is the back side (side opposite to the acoustic matching layer 34). It is preferable that the backing member 37 has an arc shape along the semi-cylindrical shape of each of the grip pieces 30a and 30b. The ultrasound oscillator 36 has a configuration in which an electrode layer 38a and an electrode layer 38b formed in a plate shape thinner than a piezoelectric element 39 are laminated on both surfaces of the piezoelectric element 39 formed in a plate shape. The piezoelectric element 39 consists of a rectangular planar shape, and is polarized in a thickness direction. Therefore, a direction of the ultrasound oscillation of the ultrasound oscillator 36 and a grip direction Y in which the transducer 25 performs the grip are parallel to each other. In addition, a direction in which the ultrasound oscillator 36 oscillates is parallel to a thickness direction of the piezoelectric element 39. The stress concentration structure 31 is fixed to the acoustic matching layer 34 of at least one of the grip piece 30a or the grip piece 30b. In Fig. 5, the grip piece 30b is provided on the grip piece 30b side.

The acoustic matching layer 34 is provided in order to match the acoustic impedance between a human body of a patient and the ultrasound oscillator 36. The acoustic matching layer 34 is superimposed on the outside of the ultrasound oscillator 36, strictly speaking, on a side facing the structure S gripped by the transducer 25 with respect to the ultrasound oscillator 36. The acoustic matching layer 34 can increase the transmittance of ultrasound.

As a material for the acoustic matching layer 34, various organic materials of which the acoustic impedance having a value closer to a value of the human body as compared to the piezoelectric element 39 can be used. Specific examples thereof include an epoxy resin, silicon rubber, polyimide, and polyethylene. In addition, the acoustic matching layer 34 is formed of a plurality of layers, and a material and the number of constituent layers are selected as appropriate in accordance with the value of required acoustic impedance.

An air gap layer 40, which is a gap capable of reflecting ultrasound with internal air, is formed between the electrode layer 38b on an outer surface side of the ultrasound oscillator 36 having the plate shape and the backing member 37 having the arc shape. As the air gap layer 40, a preferable material from the viewpoint of heat and oscillation need only be selected.

It is preferable that the piezoelectric element 39 having a mechanical quality coefficient Qm of 500 or more is used. The mechanical quality coefficient Qm is a coefficient representing an elasticity loss caused by the oscillation and is represented by a reciprocal of a mechanical loss coefficient. In a case in which the piezoelectric element 39 elastically oscillates, a loss occurs internally and is converted into heat.

In a case in which a temperature rise caused by heat generated in the piezoelectric element 39 exceeds 100°C, a possibility of depolarization of the piezoelectric element 39 is also considered. A Curie temperature of the piezoelectric element 39 is desirably approximately 1.5 times or more a target reach temperature of a surface of the piezoelectric element during the power supply, and preferably 2 times or more. For example, in a case in which the target reach temperature of the surface of the piezoelectric element is set to 200°C, the Curie temperature of the piezoelectric element 39 to be used is set to 300°C or more and preferably 400°C or more. It should be noted that, since there is a possibility in which protein is carbonized at a temperature higher than 200°C, the target reach temperature is preferably 250°C at its maximum.

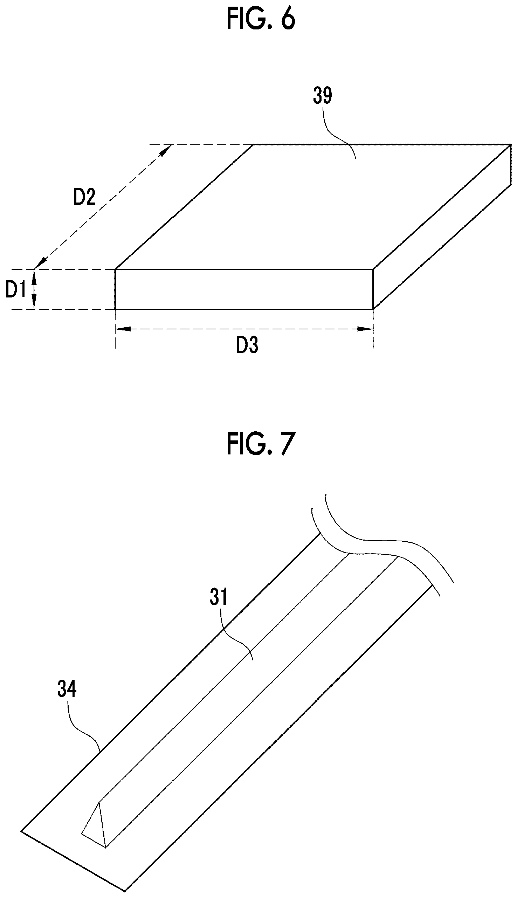

As illustrated in Fig. 6, the transducer 25 including the piezoelectric element 39 has a size that can be used for endoscopic surgery or laparoscopic surgery, and a thickness dimension D1, a length dimension D2, and a width dimension D3 of the piezoelectric element 39 alone are within a range that can be accommodated in the transducer 25. Specifically, the thickness dimension D1 is preferably in a range of 0.2 mm or more and 1 mm or less, the length dimension D2 is preferably in a range of 10 mm or more and 20 mm or less, the width dimension D3 is preferably in a range of 2 mm or more and 4 mm or less, and the thickness dimension D1 is more preferably in a range of 0.4 mm or more and 0.6 mm or less.

In a case in which the thickness of the piezoelectric element 39 is thick, heat generated on the surface side of the piezoelectric element 39 not being in contact with the biological tissue is unlikely to be transmitted to the biological tissue compared to the surface side in contact with the biological tissue, and a temperature difference between both surfaces tends to be large. On the other hand, in a case in which the thickness of the piezoelectric element 39 is thin, an amount of heat generated by the entire piezoelectric element is reduced. A thickness resonance frequency of the piezoelectric element 39 in a state of being accommodated in the transducer 25 depends on the thickness of the piezoelectric element 39, and the frequency tends to be higher as the piezoelectric element 39 is thinner. In addition, a length resonance frequency of the piezoelectric element 39 in a state of being accommodated in the transducer 25 depends on the length of the piezoelectric element 39, and the frequency tends to be higher as the piezoelectric element 39 is shorter.

Further, in the piezoelectric element 39, a member that holds one surface of the piezoelectric element 39 is held by the transducer housing via the backing member 37 having a low thermal conductivity, and a surface on the side in contact with a biological tissue is covered with the acoustic matching layer 34 consisting of a member having a high thermal conductivity or a surface protective layer having a high thermal conductivity. Here, it is also effective to provide the air gap layer 40 in a part between the ultrasound oscillator 36 and the backing member 37 to more effectively reduce heat transfer properties.

In addition, a difference in thermal expansion coefficient between the piezoelectric element 39 and the acoustic matching layer 34 generates internal stress between the ultrasound oscillator 36 and the acoustic matching layer 34 during the temperature rise, and is a factor of variation in temperature dependence of the thickness resonance frequency and the length resonance frequency of the piezoelectric element 39 described later, so that the acoustic matching layer 34 is selected in consideration of the thermal expansion coefficient of the piezoelectric element 39.

The electrode layers 38a and 38b are connected to the drive device 22 via a signal cable (not illustrated) that constitutes a power supply circuit. The signal cable is provided in a flexible tubular sheath 21a. For example, the signal cable is wired along an inner peripheral surface or an outer peripheral surface of the flexible tubular sheath 23. In a case in which the surgical treatment tool 21 is connected to the drive device 22, the electrode layers 38a and 38b are electrically connected to the drive device 22 via the signal cable. It should be noted that one of the electrode layer 38a and the electrode layer 38b is connected to a ground via the signal cable or the like, and power of an alternating current voltage signal, which will be described later, is supplied from the drive device 22 to the other thereof.

As illustrated in Fig. 7, the stress concentration structure 31 has, for example, a structure in which a side surface of a triangular prism that is a rectangular shape is a bottom surface and a wedge-shaped distal end part faces an inner side in the grip direction Y, and is preferably fixed to the surface of the acoustic matching layer 34 so as to be parallel to the longitudinal direction of the grip part 30. It is preferable that one end of the triangular prism is located in the vicinity of the distal end of the transducer 25, and the other end is linearly disposed toward the opening/closing mechanism 32 and is parallel to the longitudinal direction of the transducer. The method of fixing the stress concentration structure 31 to the acoustic matching layer 34 is not particularly limited. It should be noted that it is preferable that the stress concentration structure 31 has a shape that does not incise the structure S during the pressurized grip, specifically, a shape in which a distal end part of a wedge shape in which the stress is concentrated is not too sharp and a bottom surface is not too high with respect to the structure S. The stress concentration structure 31 is not limited to a linear shape, and may have a dotted line shape or the like as long as the structure can concentrate the stress.

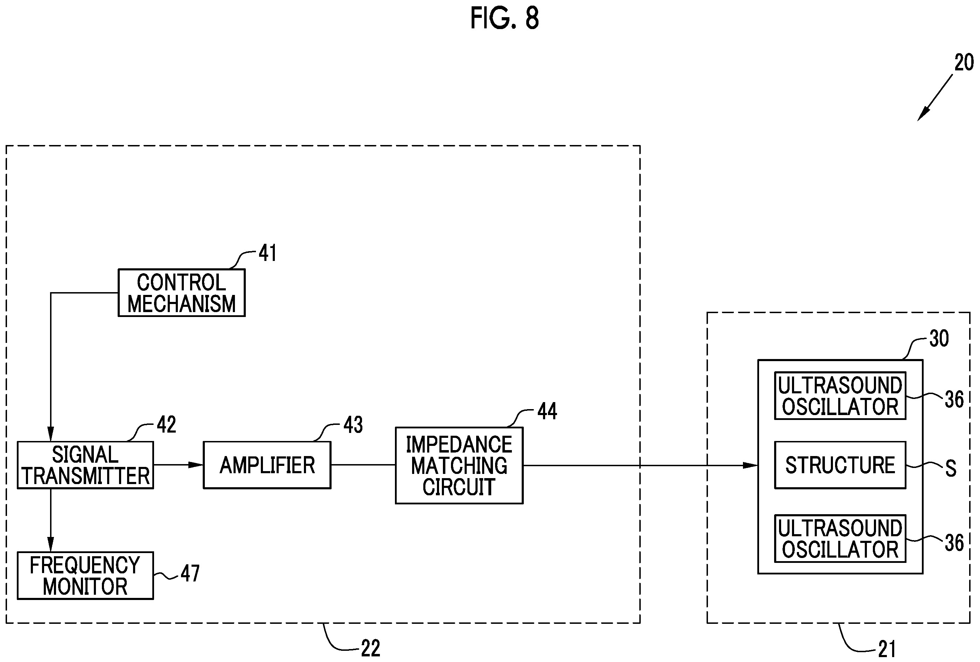

As illustrated in Fig. 8, the drive device 22 constituting the surgical treatment device 20 comprises a control mechanism 41, a signal transmitter 42, an amplifier 43, an impedance matching circuit 44, and a frequency monitor 47, and the impedance matching circuit 44 is connected to the ultrasound oscillator 36 including the piezoelectric element 39 of the transducer 25 in the surgical treatment tool 21. The control mechanism 41 controls the amount of the power supplied to the piezoelectric element 39, and a path connecting the signal transmitter 42, the amplifier 43, the impedance matching circuit 44, and the ultrasound oscillator 36 includes the power supply circuit for driving the piezoelectric element 39. It should be noted that the number of the amplifiers 43 and the impedance matching circuits 44 may be provided in accordance with the type of the driving frequency to be used.

A program related to various types of processing is stored in a program memory (not illustrated) of the drive device 22. The control mechanism 41 configured by a processor controls the functions of the signal transmitter 42, the amplifier 43, and the impedance matching circuit 44 by executing the program in the program memory.

The frequency actually used in a case of driving the ultrasound oscillator 36 is displayed on the frequency monitor 47. The signal transmitter 42 has a function of generating the alternating current voltage signal having any frequency and waveform and has, for example, the same configuration and function as a known function generator. It should be noted that it is preferable that the drive device 22 is provided with a current probe (not illustrated) and a current value monitor (not illustrated) configured by an oscilloscope or the like.

The control mechanism 41 recognizes the start of the grip in the surgical treatment tool 21, and controls the power supply at two types of driving frequencies in response to the start of the grip. In addition, it is preferable that the control of the grip is also performed together with the control of the power supply. In the control of the two types of driving frequencies, for example, after the driving at one frequency for a preset time, the driving frequency is automatically switched to the other frequency. It also controls whether the grip is maintained or is released.

The signal transmitter 42 outputs the alternating current voltage signal having a predetermined frequency. The predetermined frequency is a frequency at which the ultrasound oscillation of the piezoelectric element 39 is maximized, that is, the resonance frequency. The piezoelectric element 39 has the resonance frequency derived from the thickness dimension D1 illustrated in Fig. 6, and, specifically, in a case in which the thickness resonance frequency corresponding to the thickness dimension D1 (m) is denoted by f1 (MHz) and the piezoelectric acoustic velocity (the velocity of the acoustic wave transmitted through the piezoelectric element 39) is denoted by v (m/sec), a relationship represented by D1 = v/2f1 is satisfied. It is preferable that the frequency of the thickness resonance frequency f1 is in a range of 1 MHz or more and 10 MHz or less. It should be noted that the thickness resonance frequency f1 may shift depending on constraint conditions such as a layer configuration of the piezoelectric device or fixation to the backing material, and the thickness resonance frequency in the actual device is a frequency at which the impedance has a local minimum in the vicinity of f1.

In addition, the piezoelectric element 39 has the resonance frequency derived from the length dimension D2 illustrated in Fig. 6, and, specifically, in a case in which the thickness resonance frequency corresponding to the length dimension D2 (m) is denoted by f2 (kHz) and the piezoelectric acoustic velocity is denoted by v (m/sec), a relationship represented by D2 = v/2f2 is satisfied. It is preferable that the frequency of the length resonance frequency f2 is in a range of 1 kHz to 1 MHz. It should be noted that the length resonance frequency f2 may shift depending on constraint conditions such as the layer configuration of the piezoelectric device or fixation to the backing material, and the length resonance frequency in the actual device is a frequency at which the impedance has a local minimum in the vicinity of f2.

The signal transmitter 42 outputs the alternating current voltage signal of the driving frequency, for example, the thickness resonance frequency f1 and the length resonance frequency f2 to the amplifier 43. The amplifier 43 amplifies the alternating current voltage signal output from the signal transmitter 42 to a voltage of a level at which the ultrasound oscillator 36 can be driven. The impedance matching circuit 44 is connected in series to the amplifier 43 and can match the input impedance of the alternating current voltage signal output from the amplifier 43 with the impedance of the ultrasound oscillator 36.

The current probe measures a current value input from the impedance matching circuit 44 to the ultrasound oscillator 36, and inputs the current value to the control mechanism 41. Then, the current value is displayed on the current value monitor. The control mechanism 41 controls the signal transmitter 42 so that the current value measured by the current probe drives the ultrasound oscillator 36. The control mechanism 41 controls the signal transmitter 42 so that the alternating current voltage signal for driving the ultrasound oscillator 36 is continuously supplied. The term "continuously supplied" herein means that the alternating current voltage signal is continuously output from the signal transmitter 42 without interruption at least during the driving of the ultrasound oscillator 36. The sealing and incision treatments by the surgical treatment tool 21 are performed by using the driving of the ultrasound oscillator 36.

The ultrasound oscillation efficiency of the transducer at the thickness resonance frequency f1 and the length resonance frequency f2 is optimized by the impedance matching circuit 44. On the other hand, in a case of the sealing and incision treatments for the structure S such as the blood vessel, the transducer performance required is important in terms of thermal energy as well as ultrasound energy. Therefore, the transducer 25 is required to implement the driving method of driving the transducer 25 under the condition in consideration of the output of the ultrasound energy and the thermal energy, that is, the self-heating of the piezoelectric element 39. The control of the amount of heat generated by the piezoelectric element 39 is implemented by adjusting a voltage or a current in a power supply circuit.

The temperature dependence of the thickness resonance frequency f1 and the length resonance frequency f2 of the transducer 25 may increase due to the surrounding environment of the piezoelectric element 39, that is, interference with the backing member 37, the acoustic matching layer 34, and the like. In this case, each frequency is set in consideration of the target reach temperature range. Specifically, in a case in which the thickness resonance frequency f1 of the transducer 25 fluctuates in a range from a treatment start temperature to the target reach temperature, the frequency need only be set to a frequency that satisfies an allowable range of the thickness resonance frequency f1 in each temperature range between the treatment start temperature and the target reach temperature of the transducer 25. The same applies to the length resonance frequency f2.

In the present embodiment, an example will be described in which the sealing and incision are performed by using the surgical treatment device 20 in a case in which the structure S, which is the biological tissue, is the blood vessel. The surgical treatment device 20 comprises the transducer 25 including the piezoelectric element 39, the impedance matching circuit 44 for driving the piezoelectric element 39, the power supply wiring line that supplies the power to the piezoelectric element 39, and the control mechanism 41 of the amount of the power supplied to the piezoelectric element 39, and the transducer 25 provided at the distal end of the surgical treatment tool 21 performs the sealing and incision by using the piezoelectric element 39 provided in at least one of the grip piece 30a and the grip piece 30b and the stress concentration structure 31 provided in at least one of the grip piece 30a and the grip piece 30b. The transducer 25 includes the opening/closing mechanism 32 that opens and closes the grip pieces 30a and 30b that grip the biological tissue, and the impedance matching circuit 44 drives the piezoelectric element 39 by using the first driving frequency during the sealing and by using the second driving frequency during the incision, the first driving frequency and the second driving frequency being frequencies different from each other.

The control mechanism 41 switches between a normal mode before the structure S such as the blood vessel is gripped, a thickness direction resonance mode in which driving is performed at the first driving frequency during the pressurized grip and sealing, and a length direction resonance mode in which driving is performed at the second driving frequency during the pressurized grip and incision. C-213 (manufactured by FUJI CERAMICS CORPORATION) having the thickness dimension D1 of 0.5 mm, the length dimension D2 of 20 mm, and the width dimension D3 of 3 mm is employed as the piezoelectric element 39.

It is preferable that the thickness resonance frequency f1 is mainly used as the first driving frequency, and it is preferable that the length resonance frequency f2 is mainly used as the second driving frequency. The thickness resonance frequency f1 is the resonance frequency derived from the thickness dimension D1 of the piezoelectric element 39, and the length resonance frequency f2 is the resonance frequency derived from the length dimension D2 of the piezoelectric element 39. The impedance matching circuit 44 controlled by the control mechanism 41 of the drive device 22 is used for the mode switching. It should be noted that the actual driving frequency does not need to be strictly the thickness resonance frequency f1 or the length resonance frequency f2.

It is preferable that the control mechanism 41 has a function of detecting the grip, and automatically switches and performs the heating sealing and the incision driving on the gripped structure S. The detection of the grip is the detection that the grip pieces are closed with a certain strength or more in the closing operation in the opening/closing operation of the transducer performed by the user. Therefore, a grip detection unit (not illustrated) that detects the grip strength is provided in the grip part 30 or the opening/closing mechanism 32, and the detected grip strength is transmitted to the control mechanism 41.

The mode is automatically switched from the normal mode to the thickness direction resonance mode in response to the detection of the grip in the grip pieces 30a and 30b provided in the transducer 25, the driving at the first driving frequency is started, and the sealing treatment including the blood vessel sealing by the piezoelectric heating sealing is executed in a first set period set in advance. The first set period is a period, which is required for the sealing treatment of the structure S and is set in advance by the user, and is, for example, 5 seconds or 10 seconds. It should be noted that, during the period of the sealing treatment, a temperature is preferably a temperature after the ultrasound oscillator 36 having the piezoelectric element 39 has been heated. In a case in which the structure S is a biological tissue other than the blood vessel, a cauterization treatment or a coagulation treatment may be performed without being limited to the sealing treatment.

In the thickness direction resonance mode, the transducer 25 including the piezoelectric element 39 grips the biological tissue, and the self-heating of the piezoelectric element 39 is conducted to the biological tissue with the start of the predetermined power supply, and the ultrasound oscillation generated as an accompaniment is applied, so that the sealing of the biological tissue is effectively performed by the synergistic effect of heat, oscillation, and the grip pressure. In the thickness direction resonance mode, the power consumption contributing to the self-heating in the piezoelectric element 39 is made larger than the power consumption contributing to the ultrasound oscillation, and the impedance adjustment using the impedance matching circuit 44 is used to implement the thickness direction resonance mode.

In a case of the sealing of the blood vessel by the driving at the first driving frequency, it is preferable that the blood vessel is pressurized more than at the start of the grip. In a case of the heating and sealing, the blood vessel at the gripped part is pressurized to remove moisture and to be narrowed. As a result, it is possible to more effectively perform the incision in the length direction resonance mode. It should be noted that the grip is automatically controlled to be maintained or released, but the grip strength on the structure S can be controlled in a stepwise manner by the user operation through the operating part 24. It is preferable that the control is not control using a program or the like, but control using a mechanical structure.

The thickness direction resonance mode is a mode in which the driving conditions for actively promoting the self-heating of the piezoelectric element 39 are optimized, and the piezoelectric element 39 is driven at the thickness resonance frequency f1, and the power consumption contributing to the self-heating is larger than the power consumption contributing to the ultrasound oscillation. The power consumption contributing to the self-heating of the transducer 25 is a difference between the total power consumption and the power consumption contributing to the generation of the ultrasound oscillation.

With the end of the first set period, the mode is automatically switched from the thickness direction resonance mode to the length direction resonance mode, driving at the second driving frequency is started, and the incision treatment is executed in a second set period set in advance. The second set period is a period, which is required for the incision of the structure S, which has been subjected to the sealing treatment, and is set in advance by the user, and is, for example, 3 seconds or 5 seconds. In addition, the grip of the blood vessel by the grip part 30 (transducer 25) is controlled by the control mechanism 41, and the grip is maintained before and after the switching to the length direction resonance mode. It should be noted that while the grip is maintained, the grip strength may be changed, that is, the grip condition may be changed between the sealing and the incision.

The length direction resonance mode is a mode in which a driving condition for actively promoting the ultrasound oscillation of the piezoelectric element 39 is optimized, and the piezoelectric element 39 is driven at the length resonance frequency f2, and the power consumption contributing to the ultrasound oscillation is larger than the power consumption contributing to the self-heating. The incision treatment is performed in which the ultrasound oscillation is transmitted to the blood vessel that has been subjected to the sealing treatment with the stress through the stress concentration structure 31, and the incision is performed. It should be noted that the pressurization may be further performed in the gripping state during the second set period.

After the end of the second set period, the grip is released, and the treatment on the blood vessel ends. The switching from the length direction resonance mode to the normal mode is performed in response to the end of the treatment. The surgical treatment device 20 in the normal mode may specify the structure S such as the blood vessel as another treatment target together with the endoscope system 10, and may perform the treatment in the thickness direction resonance mode and the length direction resonance mode in the same manner. It should be noted that, in a case in which the blood vessel is excised, the blood vessel may be resected by performing the sealing and incision treatments twice. In this case, one end to be excised is sealed and incised in the first treatment, the other end is sealed and incised in the second treatment, and the target blood vessel is excised from the subject.

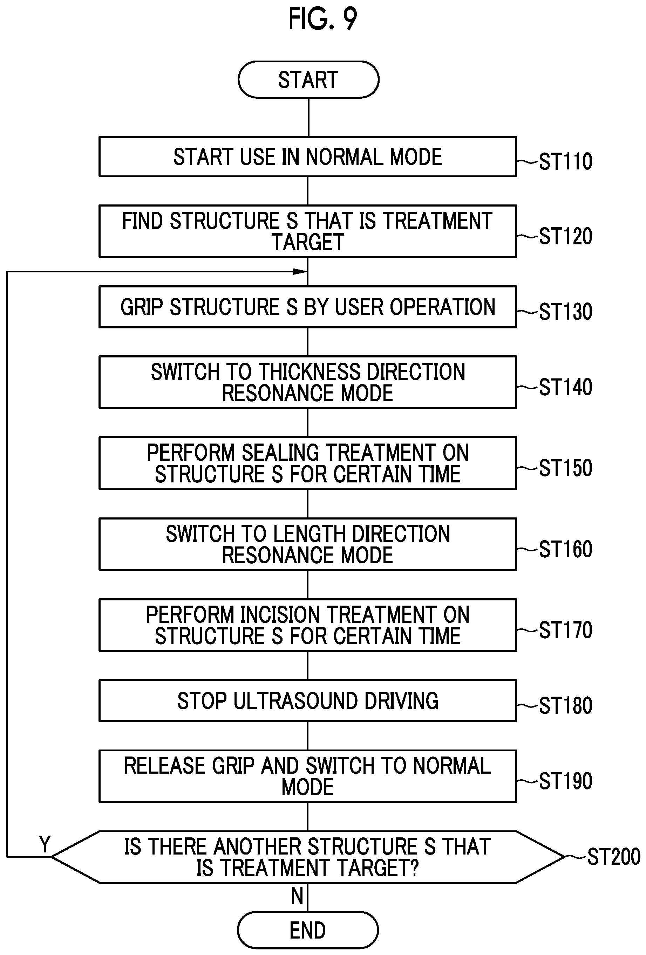

Next, the series of flow of the sealing and incision of the structure S, which is the biological tissue such as the blood vessel, by using the surgical treatment device 20 will be described with reference to the flowchart illustrated in Fig. 9. The surgical treatment device 20 starts using the surgical treatment tool 21 to be inserted into the subject through the endoscope 12 in the normal mode (step ST110). The user observes the inside of the subject using the endoscope system 10 comprising the surgical treatment tool 21 and finds the structure S that is the treatment target (step ST120). The user operates the operating part 24 of the surgical treatment tool 21 to grip the structure S using the transducer 25 (step ST130). The control mechanism 41 in the drive device 22 detects the grip and switches the normal mode to the thickness direction resonance mode (step ST140). In the thickness direction resonance mode, the first driving frequency at which the self-heating is larger than the ultrasound oscillation is allowed to flow into the ultrasound oscillator 36, and the sealing treatment of heating and sealing the structure S is performed for a certain time (step ST150). It should be noted that, in the thickness direction resonance mode, the sealing treatment is performed in a state in which the pressurized grip is automatically maintained instead of the user operation.

After the heating sealing for a certain time, the grip is automatically switched from the thickness direction resonance mode to the length direction resonance mode in a state in which the grip is maintained (step ST160). In the length direction resonance mode, the second driving frequency at which the ultrasound oscillation is larger than the self-heating is allowed to flow into the ultrasound oscillator 36, and the incision treatment of incising the structure S is performed for a certain time (step ST170). After the incision treatment for a certain time, the ultrasound driving is automatically stopped (step ST180). The surgical treatment device 20 in which the ultrasound driving is stopped switches from the length direction resonance mode to the normal mode (step ST190). In a case in which there is another structure S that is the treatment target in the subject (Y in step ST200), a new structure S is gripped by the user operation, and the sealing treatment and the incision treatment are performed (step ST130). In a case in which there is no structure S that is the treatment target (N in step ST200), the use of the surgical treatment device 20 ends.

With the above-described configuration, in the structure S which is the biological tissue such as the blood vessel, a necessary part can be sealed by the self-heating, and the sealed part can be accurately incised by the ultrasound oscillation. The thickness direction resonance mode and the length direction resonance mode automatically proceed by detecting the grip of the structure S, so that the sealing and incision can be performed without depending on the skill of the user or the like.

In the present embodiment, the ultrasound oscillator 36 is provided in the pair of grip pieces 30a and 30b constituting the transducer 25, but the present invention is not limited thereto, and the ultrasound oscillator 36 may be provided in only one of the grip piece 30a or the grip piece 30b that forms a pair. In this case, it is preferable that the ultrasound oscillator 36 is disposed, for example, parallel to the inner surface of one grip piece 30a, and the inner surface of the other grip piece 30b facing the ultrasound oscillator 36 is made of a material that reflects the ultrasound oscillation of the ultrasound oscillator 36. In addition, the stress concentration structure 31 may be provided on the inner surface of the grip pieces 30a and 30b on which the ultrasound oscillator 36 is provided, may be provided on the other inner surface, or may be provided on both the inner surfaces.

In the present embodiment, the hardware structure of the processing units that execute various types of processing, such as the control mechanism 41, is the following various processors. The various types of processors include a central processing unit (CPU) that is a general-purpose processor functioning as various types of processing units by executing software (program), a graphical processing unit (GPU), a programmable logic device (PLD) that is a processor of which a circuit configuration can be changed after manufacture, such as a field programmable gate array (FPGA), and a dedicated electric circuit that is a processor of which a circuit configuration is specifically designed to execute various types of processing.

One processing unit may be configured by one of the various processors, or may be configured by a combination of two or more processors of the same type or different types (for example, a plurality of FPGAs, a combination of a CPU and an FPGA, or a combination of a CPU and a GPU). Also, a plurality of the processing units may be configured by one processor. As an example in which the plurality of processing units are configured by one processor, first, there is a form in which one processor is configured by a combination of one or more CPUs and software, and this processor functions as the plurality of processing units, as represented by a computer, such as a client or a server. Second, there is a form in which a processor, which implements the functions of the entire system including the plurality of processing units with one integrated circuit (IC) chip, is used, as represented by a system-on-a-chip (SoC) or the like. In this way, various processing units are configured by one or more of the various processors described above, as the hardware structure.

Further, the hardware structure of these various processors is, more specifically, an electric circuit (circuitry) having a form in which circuit elements such as semiconductor elements are combined. In addition, the hardware structure of the storage unit is a storage device, such as a hard disc drive (HDD) or a solid-state drive (SSD).

In addition, in the above-described embodiment, the endoscope 12 to be combined with the surgical treatment tool 21 according to the embodiment of the present invention is not specified, but any endoscope may be used as long as the endoscope comprises the forceps channel into which the treatment tool is inserted, and may be, for example, a bronchoscope, an upper gastrointestinal endoscope, or a lower gastrointestinal endoscope. It is preferable that the surgical treatment device 20 including the impedance matching circuit 44 according to the present embodiment is implemented at the time of manufacturing in a factory or the like.

### Explanation of References

10: endoscope system
12: endoscope
12a: insertion part
12b: operating part
12c: bendable part
12d: distal end part
12e: angle knob
12f: forceps port
12g: forceps channel
12h: forceps outlet
14: light source device
15: processor device
16: display
17: user interface
20: surgical treatment device
21: surgical treatment tool
22: drive device
23: flexible tubular sheath
24: operating part
25: transducer
30: grip part
30a: grip piece
30b: grip piece
31: stress concentration structure
32: opening/closing mechanism
33: support member
34: acoustic matching layer
35: operation wire
36: ultrasound oscillator
37: backing member
38a: electrode
38b: electrode
39: piezoelectric element
40: air gap layer
41: control mechanism
42: signal transmitter
43: amplifier
44: impedance matching circuit
47: frequency monitor
D1: thickness dimension
D2: length dimension
D3: width dimension
S: structure
Y: grip direction

## Claims

1. A surgical treatment device comprising:
a treatment tool including a transducer including a piezoelectric element;
an impedance matching circuit for driving the piezoelectric element;
a power supply wiring line that supplies power to the piezoelectric element; and
a control mechanism that controls an amount of the power supplied to the piezoelectric element,
wherein the transducer is located at a distal end of the treatment tool and includes an opening/closing mechanism that opens and closes grip pieces that grip a biological tissue,
the piezoelectric element is installed on at least one of the grip pieces,
a stress concentration structure is provided on at least one of the grip pieces,
the impedance matching circuit drives the piezoelectric element by using a first driving frequency and a second driving frequency that are frequencies different from each other, and
the piezoelectric element is driven by using the first driving frequency in a case in which the biological tissue is sealed, and is driven by using the second driving frequency in a case in which the biological tissue is incised.

2. The surgical treatment device according to claim 1,
wherein the first driving frequency is a resonance frequency derived from a thickness dimension of the piezoelectric element, and the second driving frequency is a resonance frequency derived from a length dimension of the piezoelectric element.

3. The surgical treatment device according to claim 2,
wherein a frequency of the first driving frequency is in a range of 1 MHz or more and 10 MHz or less, and a frequency of the second driving frequency is in a range of 1 kHz or more and 1 MHz or less.

4. The surgical treatment device according to claim 1,
wherein power supply at the first driving frequency is executed in a first set period in response to detection of the grip of the transducer.

5. The surgical treatment device according to claim 1,
wherein power supply at the second driving frequency is executed in a second set period, and
the grip is released in response to an end of the second set period.

6. The surgical treatment device according to claim 1,
wherein switching from the first driving frequency to the second driving frequency is executed in a state in which the grip is maintained.

7. The surgical treatment device according to claim 6,
wherein the switching from the first driving frequency to the second driving frequency is automatically executed in response to an end of power supply at the first driving frequency.

8. The surgical treatment device according to claim 1,
wherein pressurization is performed during power supply at the first driving frequency, and a grip strength of the transducer is stronger than a grip strength at a start of the grip.

9. The surgical treatment device according to claim 1,
wherein a grip strength of the transducer is controlled in a stepwise manner by a manual operation of a user.

10. The surgical treatment device according to claim 1,
wherein the stress concentration structure is linearly disposed from a vicinity of a distal end of the transducer toward the opening/closing mechanism, and is parallel to a longitudinal direction of the transducer.
